Europäisches Patentamt

European Patent Office

Office européen des brevets

(19)

(11) Publication number: **0 114 614**
**A2**

(12) # EUROPEAN PATENT APPLICATION

(21) Application number: **84100249.6**

(22) Date of filing: **12.01.84**

(51) Int. Cl.³: **G 01 N 33/74**
**G 01 N 33/54**

(30) Priority: **25.01.83 US 460739**

(43) Date of publication of application:
**01.08.84 Bulletin 84/31**

(84) Designated Contracting States:
**BE DE FR GB IT**

(71) Applicant: **ABBOTT LABORATORIES**
**14th Street and Sheridan Road**
**North Chicago, Illinois 60064(US)**

(72) Inventor: **March, Steven Carl**
**1307 Winchester Road**
**Libertyville Illinois 60048(US)**

(74) Representative: **Modiano, Guido et al,**
**MODIANO, JOSIF, PISANTY & STAUB Modiano &**
**Associati Via Meravigli, 16**
**I-20123 Milan(IT)**

(54) Determination of steroid hormone glucuronides.

(57) The present invention relates generally to the quantitative determination of steroid hormone glucuronides. More specifically, the invention provides a novel immunological assay procedure for quantifying specific steroid hormone glucuronides utilizing a tracer comprising a steroid hormone glucuronide covalently bound to an enzyme.

EP 0 114 614 A2

DETERMINATION OF STEROID HORMONE GLUCURONIDES

The present invention relates generally to the quantitative determination of steroid hormone glucuronides. More specifically, the invention provides a novel immunological assay procedure for quantifying specific steroid hormone glucuronides utilizing a tracer comprising a steroid hormone glucuronide covalently bound to an enzyme.

Steroid hormones are hormones normally synthesized from cholesterol and secreted by the adrenal cortex, testes, ovaries, placenta, and corpus leuteum of organisms. By definition, the above-mentioned steroid hormones are physiologically effective in trace amounts. See *Introduction To Modern Biochemistry*, Academic Press, New York, New York, (3rd Edition, 1971), page 358, wherein the physiological effects of several vertebrate steroid hormones are set forth as in Table I.

TABLE I

Physiological Effects Of
Vertebrate Steroid Hormones

| Steroid Hormone | Gland Producing The Hormone | Effect |
| --- | --- | --- |
| Corticosteroids | Adrenal cortex | Mineral Balance: $Na^+$ retention<br><br>Metabolism: Gluconeogenesis |
| Progesterone | Ovary (corpus leuteum) | Proliferation of uterine mucosa (secretory phase) |
| Estradiol | Ovary (follicles) | Proliferation of the uterine mucosa (estrus) |
| Testosterone | Testes (interstitial cells) | Maintenance of the accessary glands of the genital tract and secondary sex characteristics |

Inactivation of steroid hormones is achieved by metabolism in the liver or excretion in urine. Karlson, *supra,* pp. 260, 361-363. It is known that in humans endogenous steroids are excreted in urine principally as water-soluble conjugates. See, Kellie, "The Radioimmunoassay of Steroid Conjugates", *J. Steriod Biochem.,* 6, p. 277 (1975). Derivatives of the pregnane series (progesterone) are excreted almost exclusively as water-soluble glucuronides (pregnanediol glucuronide), and the principal excretory form of estrogens are also glucuronides (estradiol glucuronide, estriol glucuronide, and esterone glucuronide). Kellie, supra, p. 277.

The important physiological effects elicited by steroid hormones have prompted substantial research into methods for detection and quantification of these hormones and their glucuronide derivatives. See, Jeffcoate, "Progress Toward The Wider Use Of Steroid Immunoassays", *J. Steroid Biochem.,* 11, pp. 1051-1055 (1979).

One of the most studied relationships between endogenous concentrations of steroid hormones and their physiological effects has been role of the so-called female sex hormones (estrogen and progesterone) in the menstrual cycle. It is generally agreed that peak estrogen excretion occurs about the middle of the human female menstrual cycle, and that during the leuteal phase the pregnanediol (metabolite of progesterone) output rises and a second estrogen peak occurs. Brown, et al, "The Urinary Excretion of Oestrogens, Pregnanediol and Gonadotropins During The Menstrual Cycle", *J. Endocrin.,* 17, p. 401 (1958). A recent study suggests the determination of estradiol glucuronide levels may provide a useful indicator for the day of ovulation in humans and increased preganediol glucuronide levels may provide evidence of ovulation. See, Stanczyk, et al, "Direct Radioimmunoassay for Urinary Estrogen and Pregnanediol Glucuronides During The Menstrual Cycle", *American Journal of Obstetrics and Gynecology,* 137, p. 443 (1980). Treatment of infertility in humans has also been facilitated where urinary and plasma estradiol levels and serum progesterone levels were monitored. See, Black, et al., "An Assessment Of Urinary And Plasma Steroid Estimations for Monitoring Treatment of Anovulation with Gonodotropins", *J. Obstet. Gynec. British Commonwealth,* **81**, page 667 (1974).

Various assay methods have previously been employed to quantify steroid hormones in serum and steroid hormone glucuronides in urine samples. Although studies have been conducted using higher vertebrates (See, Seeger, et al, "An Enzyme Immunoassay (EIA) for Pregesterone in Horse Plasma", *J. Immun. Methods,* 28, page 211 (1979)), most assays have been directed to quantifying human steroid hormones or their glucuronide derivatives. Steroid hormones may occur in human body fluids in concentrations ranging from picomoles per liter (serum estradiol, early leuteal phase of menstrual cycle), to as much as 100 micromoles per liter (urinary estriol at end of pregnancy). See, van Weeman, et al, "Enzyme-Immunoassay of Steroids: Possibilities and Pitfalls", *J. Steroid Biochem.,* **11**, page 147 (1979). Although less sensitive bioassays or chemical assays may be utilized to measure steroid hormones present in high concentrations, such assays lack the necessary sensitivity for many applications.

The development of radioimmunoassay techniques has provided assays with sensitivities in the picomole per liter range. Typical radioimmunoassays involve addition of a fixed amount of radiolabelled steroid hormone to the steroid-containing sample, followed by the addition of a fixed amount of steroid antibodies, and subsequent measurement of the radioactivity of antibody-bound or unbound radiolabeled steroid hormone. See, van Weeman, *supra,* page 147.

Radioimmunoassay of serum steriod hormones require repeated venipuncture of the subject. In addition to patient reluctance to venipuncture (see, Seaton, et al, "Use of Salivary Progesterone Assays to Monitor Menstrual Cycles in Bangladeshi Women", *Clin. Chem.,* 789:1033 (1979)), trained medical personnel are generally required to take the blood samples.

Radioimmunoassays directed at determination of urinary steroid hormone concentrations have also been developed. See, Kellie, *supra,* pp. 277-281; Stanczyk, et al, "Direct Radioimmunoassay of Urinary Estrogen and Pregnanediol Glucuronides During the Menstrual Cycle", *Am. J. Obstet. Gynecol.,*

137, pp. 443-450 (1980). Steroid hormone glucuronides determined directly (without hydrolysis of glucuronide derivatives into free steroid hormone) include estrone glucuronide, estradiol-3-glucuronide, estradiol-17β-glucuronide, estriol-3-glucuronide, estriol-16α-glucuronide, and pregnanediol 3α-glucuronide. Stanczyk, et al, *supra*, pp. 443-444. Determination of steroid hormone glucuronides by radioimmunoassay of urine samples negates the need for blood sampling.

Significant problems are associated with the use of radiolabeled compounds, include the need for specially equipped laboratories, specially trained personnel, expensive radioactive counting equipment, the short half-life of some isotopes, waste-disposal problems, and possible licensing problems depending on lacal legislation. See, van Weeman, *supra*, page 147. In addition, significant time is required to complete radioimmunoassays. A recently reported "rapid" radioimmunoassay of steroid hormone glucuronides required 2-1/2 hours of incubation time prior to the radioactive counting procedure. See, Chatteron, et al, "Radioimmunoassay of Pregnanediol Concentrations In Early Morning Urine Specimens For Assessment Of Leuteal Function In Women", *Fertility and Sterility*, 37, pp. 361-363 (1982). Other rapid assays require treatment of the urine sample with hot acid to hydrolyze the steroid hormone glucuronides into free steroid hormones before performing the actual quantification step. See, Metcalf, "Rapid Gas Chromatographic Assay For Progesterone Metabolites In Urine", *Clin. Biochem.*, 6, pp. 307-308 (1973).

The use of nonisotopic steroid hormone immunoassays has been proposed to avoid many of the problems inherent in radioimmunoassays. Nonisotopic assays for steroid hormones include labeled ligand assays wherein determination of the steroid hormone consists of measuring the biological or chemical activity of fluorophores, bacteriophages, coenzymes, or enzymes. See, van Weeman, *supra*, page 147. Assays utilizing the latter approach commonly referred to as enzyme-immunoassays or enzyme-linked immunoassays, have been reported for the following steroid hormones: estrogens, progesterone, testosterone and cortisol.

Recently developed enzyme immunoassays for steroid hormones exhibit sensitivities equal or nearly equal to those of radioimmunoassays. See, Numazawa, et al., "Picogram Order Enzyme Immunoassay of Oestradiol", *FEBS Letter*, 79, pp. 396-397, (1977); Bosch, et al, "Enzyme Immunoassay for Total Oestragens in Pregnancy Plasma or Serum", *Clin. Chim. Acta.*, 89, pp. 59, 63 (1978). Chatterton, Jr., Robert T., "Radioimmunoassay Of Pregnanediol Concentrations In Early Morning Urine Specimens For Assessment of Leuteal Function In Women:, *Fertility and Sterility*, 37, (3) 361-366, (1982). These assays are generally referred to as heterogeneous enzyme-immunoassays in that the antibody-bound enzyme-labelled steroid hormone and unbound enzyme-labelled steroid hormone are both enzymatically active and must be separated before measurements are made. A common enzyme-immunoassay comprises combining enzyme-labelled steroid hormone with the steroid hormone-containing sample, incubating the mixture with anti-steroid hormone antibody, and measuring the enzymatic activity of the antibody-bound or unbound enzyme-labelled steroid hormone after separation. Enzyme-labelled steroid hormone and steroid hormone compete with each other for the available antibody binding sites. The amount of steroid hormone present in the sample is therefore related to the amount of enzyme-labelled steroid hormone bound to the antibody. See, van Weeman, supra, page 147. Enzymes conjugated to steroid hormones and utilized in enzyme immunoassays include horseradish peroxidase, β-D-galactosidase, glucoamylase, alkaline phosphatase, glucose-6-phosphatase, and malate dehydrogenase. (See van Weeman, supra, page 148.)

Although avoiding many of the problems associated with radioimmunoassay techniques, the reported steroid hormone enzyme-immunoassays all use serum samples (blood or pregnancy plasma) as their steroid hormone-containing sample. The problems inherent in obtaining multiple samples of blood or pregnancy plasma include the need for trained medical personnel and the possible subject resistance mentioned above.

- 6 -

0114614

There has been little if any progress made towards developing an enzymeimmunoassay to determine urinary steroid hormone glucuronides. This may be due in part to the lack of suitable enzyme-labeled steroid hormone glucuronide preparations. Certain enzyme-labeled steroid hormone glucuronides such as horseradish peroxidase-labeled glucuronides are generally stored in a liquid form, reducing their shelf life and increasing problems of contamination. Other enzymes show marked decreases in their enzymatic activity when covalently bound to the steroid hormone glucuronide (see, Rjaowski, et al, "The Efficiency of Different Coupling Procedures For The Linkage of Oestriol-16α-Glucuronide, Oestrone-3-Glucuronide and Pregnanediol-3α-Glucuronide to Four Different Enzymes," *J. Steroid Biochemistry*, 14, pages 861, 863-865 (1981)); or when strong noncovalent associations are used to link enzymes to other hormones. See, Guesdon, et al, "The Use Of Avidin-Biotin Interaction in Immunoenzymatic Techniques," *J. Histochem. Cytochem.*, 22, page 1131 (1979).

There exists, therefore, a long-standing need in the art for an enzyme-immunoassay to determine steroid hormone glucuronides.

Summary Of The Invention

The present invention provides a novel method for rapid, highly sensitive quantitative detection of selected steroid hormone glucuronides in a sample.

The present invention relates to a method for determining steroid hormone glucuronides in a sample, said method comprising treating the sample with a known amount of a tracer comprising the specific steroid hormone glucuronide to be determined covalently attached to an enzyme; treating the sample and tracer with a known amount of antisteriod hormone antibody to produce an assay mixture comprising antibody bound steroid hormone glucuronide, antibody, bound tracer, unbound steroid hormone glucuronide and unbound tracer; separating the unbound tracer or antibody bound tracer from the assay mixture and determining the amount of the separated antibody bound tracer or unbound tracer present as a measure of the steroid hormone glucuronide present in the sample.

Detailed Description Of The Invention

According to a preferred embodiment of the invention, the amount of steroid hormone glucuronide in a urine sample is determined by: (1) combining the sample with a known amount of a tracer comprising a ligand corresponding to the steroid hormone glucuronide to be determined covalently bound to alkaline phosphatase; (2) prior to, or simultaneously with the addition of the tracer to the sample, treating the sample with a suitable surfactant; (3) adding to the sample and tracer a known amount of antisteroid hormone glucuronide antibody to form an assay mixture comprising antibody-bound steroid hormone glucuronide, antibody-bound tracer, unbound steroid hormone glucuronide, and unbound tracer; and (4) determining the enzymatic activity of unbound tracer by precipitating antibody-bound steroid hormone glucuronide and antibody bound tracer in the assay mixture and adding a known amount of the supernate comprising unbound tracer and unbound steroid hormone glucuronide to a solution containing an alkaline phosphatase substrate.

The amounts of tracer and antisteroid hormone glucuronide antibody utilized in the method of the present invention are readily ascertained by one of ordinary skill in the art. The amount of tracer must be equal to or greater than the amount of antisteroid hormone glucuronide antibody. It is preferred that the amount of tracer employed be in excess to the amount of antisteroid hormone glucuronide antibody.

Preferred methods of the present invention utilize a tracer comprising pregnanediol glucuronide, estradiol glucuronide, estriol glucuronide, or estrone glucuronide covalently attached to alkaline phosphatase enzyme. The present invention also comprehends methods of quantitative determination of steroid hormone glucuronides wherein enzymes other than alkaline phosphatase are covalently bound to said steroid hormone glucuronides.

Another preferred embodiment of the present invention involves the determination of pregnanediol glucuronide. Pregnanediol glucuronide is a product of the enzymatic coupling of pregnanediol, a metabolite of. pregesterone, and glucuronic acid. Pregnanediol glucuronide is promptly excreted in the urine and, thus, provides a mechanism for estimating the concentration of progesterone in serum without venipuncture. Pregnanediol glucuronide, whether enzyme-linked or not, will bind to pregnanediol glucuronide specific antiserum. Therefore, the concentration of either the unlabeled or enzyme-labeled pregnanediol glucuronide in solution will determine the relative amount of binding to antibody. When pregnanediol glucuronide (unknown) from a urine specimen or a standard solution is equilibrated with a tracer comprising enzyme-labeled pregnanediol glucuronide and antipregnanediol glucuronide antibody, the amount of tracer bound to the antipregnanediol glucuronide antibody will be related to the amount of unknown present in the urine specimen or standard solution. Upon separation from the antibody bound to tracer from unbound tracer measuring the unbound tracer the concentration of pregnanediol glucuronide in the sample or standard may be determined. The amount of pregnanediol glucuronide (unknown) in the sample or standard is related to the amount of unbound tracer in the supernatant after separation from the antibody complex.

The methods of the present invention are effective in determining pregnanediol glucuronide concentrations in a range normally observed in urine, i.e., 1-40 micromoles per liter. In addition, the method of the present invention may be performed in less than one hour.

To increase the sensitivity of the methods of the present invention, it is preferred to add to the sample prior to the addition of the antisteroid hormone glucuronide antibody, a surfactant capable of maximizing the amount of free steroid hormone glucuronide in the sample. Suitable surfactants include for example, alkyl polyether sulfates, (i.e., Triton S-301, Triton X-151, Triton X-100), sodium alkyl sulfates (i.e., sodium dodecyl sulfate), quaternary amines and ammonium salts (i.e., dodecyltrimethyl ammonium bromide), cholestrol-like acids (i.e., deoxycholic acid), dioctyl sodium sulfonate (Triton GR-7M), and the like. It is preferred to employ a polyaryl sulfate as the surfactant in the methods of the present invention. It is most preferred to employ Triton X-301 as the surfactant.

As previously mentioned, the enzymatic activity of either the antibody bound tracer or unbound tracer may be determined as a measure of the steroid hormone glucuronide in the sample. Prior to the determination of the enzymatic activity, the antibody bound tracer or unbound tracer to be determined must be separated from the assay mixture. Methods for separating the antibody bound tracer or unbound tracer are readily ascertained by one of ordinary skill in the art and include for example, immunoprecipitation of antibody bound components of the assay solution, solid phase separation, and the like. It is preferred to employ an antibody specific for the antisteroid hormone glucuronide antibody to precipitate antibody bound tracer and antibody bound steroid hormone glucuronide in the assay mixture and then determine the enzymatic activity of the unbound tracer in the remaining assay mixture supernate. The enzymatic activity of the separated antibody bound tracer or unbound tracer is determined utilizing conventional techniques, such as for example, colorimetric determinations employing a substrate specific for the enzyme label.

The determination of the concentration of steroid hormone glucuronide present in the sample may be achieved by comparison of the enzymatic activity of the assay mixture supernate to a standard curve prepared utilizing an assay system employing known quantities of tracer, steroid hormone glucuronide, antisteroid hormone antibody, and alkaline phosphatase substrate.

The following examples will serve to further illustrate the present invention and are not intended to limit it in spirit or in scope.

## EXAMPLE I

In the practice of the method of the invention, the following reagents are employed: Pregnanediol glucuronide antibody solution comprised of goat antipregnanediol glucuronide suspended in a 0.1M carbonate buffer (pH 9) solution containing Triton X-301. Pregnanediol glucuronide tracer solution comprising pregnanediol glucuronide covalently attached to alkaline phosphatase enzyme, prepared according to the procedures described in Example IV or V, and suspended in carbonate buffer with protein, 1% lactose and 0.1% sodium azide; standard solutions of pregnanediol glucuronide, 0, 10, 20, 30 and 40 µM, were compiled by mixing appropriate amounts of the purified glucuronide with charcoal stripped male urine containing 0.1% sodium azide; DAPS (Double Antibody Precipitin Suspension) comprised of normal goat serum/ porcine antiboat antiserum was prepared according to Morgan and Lazarow, *Diabetes*, 12:115-126, (1963); Abbott A-Gent Alkaline Phosphatase Reagent: a quenching solution comprising 10 mM cystiene; and an unassayed urine control.

All of the above reagents may be provided in dry form and brought up to the desired volume with water immediately prior to performing the test procedure. Urine can be collected with normal home or office collection. Although steroids are stable in urine for periods of two to three days, the test procedure should preferably be performed as promptly as possible after collection.

The following example illustrates the test procedure for quantification of pregnanediol glucuronide in a urine sample.

## EXAMPLE II

The reagents of Example I are employed in the following sequence of steps: Twenty-five microliters (25 µl) of urine sample, standard solution or urine control are added to duplicate sets of culture tubes. A blank culture tube is also established by adding 25 µl of water to a separate culture tube. One hundred microliters (100 µl) of the preganediol glucuronide tracer solution is added to each culture tube except the blank tube, to which 100 µl of water is added. Fixed amounts of pregnanediol glucuronide antibody solution (100 µl) are added to all the culture tubes (including the blank), and each tube is vortexed to mix the samples thoroughly. After mixing, the tubes are covered and incubated at 37° C for ten minutes, whereupon 200 µl of DAPS is added to each culture tube. All samples are again vortexed-mixed thoroughly and incubated at 37° C for ten minutes. After incubation, all tubes are centrifuged at 1000 X g for ten minutes. One hundred microliter (100 µl) aliquots are removed from the supernate in each tube and carefully pipetted into labeled reaction tubes so as not to disturb the pellet in the bottom of each tube. To each reaction tube is added 100 µℓ of Abbott A-Gent Alkaline Phosphatase Reagent, and the samples are mixed thoroughly and incubated at room temperature for 15 minutes. After the room temperature incubation, 1.5 ml of quenching solution is added to each reaction tube and the absorbance of the samples are then read on a spectrophotometer at an appropriate wavelength for ρ-nitrophenol and compared to a standard curve prepared using a standard solution of pregnanediol glucuronide.

## EXAMPLE III

Another embodiment of the method of the present invention utilizes an immune precipitant comprising a suspension of rabbit anti-pregnanediol glucuronide antibody and goat-anti-rabbit antibody (immune DAPS). This suspension is prepared in accordance with a method analogous to the procedure employed to prepare the goat antirabbit/normal rabbit IgG (DAPS) suspension in Example I, except the binding of the alkaline phosphatase labeled pregnanediol glucuronide tracer is determined at various antibody dilutions. The antipregnanediol glucuronide precipitin is employed in the methods of the present invention in accordance with the following procedure: Twenty-five microliters (25 µℓ) of urine sample, standard solution or urine control are added to duplicate sets of culture tubes. A blank culture tube is also established by adding 25 µℓ of water to a separate culture tube. One hundred microliters (100 µℓ) of an antipregnanediol glucuronide immune DAPS precipitin suspension is added to each culture tube, including the blank, and each tube is vortexed to mix the samples thoroughly. After mixing, the tubes are covered and incubated at 37° C for ten minutes. After incubation, all tubes are centrifuged at 1000 X g for ten minutes. One hundred microliter (100 µℓ) aliquots are removed from the supernate in each tube and carefully pipetted into labeled reaction tubes so as to not disturb the pellet in the bottom of each tube. To each reaction tube is added 100 µℓ of A-Gent Alkaline Phosphatase Reagent, and the samples are mixed thoroughly and incubated at room temperature for 15 minutes. After the room temperature incubation, 1.5 ml of quenching solution is added to each reaction tube and the samples mixed thoroughly. The absorbance of the samples are then read on a spectrophotometer at an appropriate wavelength for ρ-nitrophenol and then compared to a standard curve using standard solution of pregnanediol glucuronide.

Tracers employed in the methods of the present invention may be prepared in accordance with the following procedure: an N-hydroxysuccinimide ester of pregnanediol glucuronide is prepared by mixing pregnanediol glucuronide with N-hydroxysuccinimide in the presence of 1-ethyl-3(3-dimethylaminopropyl)carbodiimide hydrochloride in dimethyl formamide. The pregnanediol glucuronide ester thus prepared is reacted with alkaline phosphatase in an appropriate buffer at molar ratios of glucuronide to alkaline phosphatase in the range of from about 500:1 to about 100:1, preferably from 200:1 to 100:1. The alkaline phosphatase labeled pregnanediol glucuronide produce is purified by gel chromatography or dialysis. Other alkaline phosphatase labeled gluruconides may be prepared by utilizing other glucuronides such as for example, estradiol, glucuronide, estriol glucuronide or esterone glucuronide, in lieu of pregnanediol glucuronide in the above-described procedure.

The following Examples serve to further illustrate the preparation of specific tracers useful in the methods of the present invention.

EXAMPLE IV

A mixture comprising pregnanediol glucuronide (100 mg, 200 μmol), N-hydroxysuccinimide (30 mg, 260 μmol), 1-hydroxybenztriazole hydrate (0.1 mg, 0.75 μmol), and N,N'-dicyclohexylcarbodiimide (57 mg, 276 μmol) was stirred in 1 ml of dry dimethylformamide for one hour at 5° C, allowed to stand at room temperature for 18 hours, filtered and the filtrate evaporated under reduced pressure to yield a residue. The residue was dissolved in dry acetone, precipitated with ether, and the precipitate collected on a filter and dried to yield a pregnanediol glucuronide active ester as a white powder. 3.2 mg of the pregnanediol glucuronide active ester was dissolved in 200 µℓ of dry dimethyl formamide. Portions of the resulting solution in molar ratios of 120:1, 145:1, and 165:1 of active ester to alkaline phosphatase were mixed with 50 µℓ of purified alkaline phosphatase and shaken for 18 hours at 4° C to yield a crude product. The crude product was purified over gel-filtration minicolumns to yield an alkaline phosphatase-labeled pregnanediol glucuronide tracer.

## EXAMPLE V

Pregnanediol glucuronide (25.0 mg, 50 µmol), N-hydroxysuccinimide (8.5 mg, 74 µmol), and 1-ethyl-3(3-dimethylaminopropyl)carbodiimide hydrochloride (815 mg, 45 µmol) were stirred in 1 ml of dry dimethyl formamide for 24 hours to yield a solution containing a pregnanediol glucuronide active ester. A portion of the solution having a molar ratio of 490:1 of pregnanediol glucuronide active ester to alkaline phosphatase was reacted with 400 µℓ of purified alkaline phosphatase to yield a crude product which was purified over 25 minicolumns and dialyzed against buffer containing 0.1M sodium phosphate, 0.1 mM $MgCl_2$ and 0.1% deoxycholic acid (pH 7.0) (2 X 500 ml) to yield an alkaline phosphatase-labeled pregnanediol glucuronide tracer.

## EXAMPLE VI

A mixture comprising estradiol glucuronide sodium salt (20 mg, 42 µmol), N-hydroxysuccinimide (6 mg, 52 µmol), and 1-ethyl-3(3-dimethylaminopropyl)carbodiimide hydrochloride (8 mg, 42 µmol) was stirred in 1 ml of dry dimethylformamide for four hours to yield an estradiol glucuronide active ester. The estradiol glucuronide active ester was reacted *in situ* with 40 µℓ of purified alkaline phosphatase in molar ratios of 60:1, 120:1, 180:1, 240:1, 300:1, and 360:1 of active ester to alkaline phosphatase to yield a crude product. The crude product was purified over minicolumns to yield an alkaline phosphatase-labeled estradiol glucuronide tracer. ·

Although the invention has been described with respect to specific modifications, the details thereof are not to be construed as limitations, for it will be apparent that various equivalents, changes and modifications may be resorted to without departing from the spirit and scope thereof and it is understood that such equivalent embodiments are intended to be included herein.

CLAIMS:

1.   A method for determining a steroid hormone glucuronide in a urine sample, said method comprising:

(a)  combining said sample with a known amount of a tracer comprising the specific steroid hormone glucuronide to be determined covalently bound to an enzyme;

(b)  treating the sample and tracer with a known amount of antisteroid hormone antibody to form an assay mixture comprising antibody-bound steroid hormone glucuronide, antibody-bound tracer, unbound steroid hormone glucuronide and unbound tracer;

(c)  separating the unbound tracer or antibody bound tracer from the assay mixture; and

(d)  determining the amount of the separated antibody bound tracer or unbound tracer present as a measure of the steroid hormone glucuronide in the sample.

2.   A method according to Claim 1 wherein the tracer comprises the specific steroid hormone glucuronide to be determined covalently attached to alkaline phosphatase.

3.   A method according to Claim 2 wherein the steroid hormone glucuronide to be determined is selected from the group consisting of pregnanediol glucuronide, estradiol glucuronide, estratriol glucuronide  and estrone glucuronide.

4.   A method according to Claim 2 and further including the step of treating said sample with a suitable surfactant prior to or simultaneously with step (b).

5.   A method according to Claim 2 wherein step (c) includes the precipitating antibody-bound tracer and antibody-bound steroid hormone glucuronide using an antibody to said antisteroid hormone antibody.

6. A method according to Claim 5 wherein un-bound tracer is determined as a measure of the steroid hormone glucuronide in the sample.

7. A method according to Claim 6 wherein the unbound tracer is colorimetrically determined.

8. A method according to Claim 5 wherein antibody bound tracer is determined as a measure of the steroid hormone glucuronide in the sample.

9. A method according to Claim 8 wherein the antibody bound tracer is colorimetrically determined.

10. A method according to Claim 2 wherein steps (b) and (c) are performed simultaneously by treating the sample and tracer with a known amount of a complex consisting of the antisteroid hormone antibody and an antibody to the anti-steroid hormone antibody, thereby resulting in the precipitation of antibody-bound tracer and antibody-bound steroid hormone glucuronide.